Europäisches Patentamt

European Patent Office

Office européen des brevets

(11) Veröffentlichungsnummer : **0 458 930 B1**

(19)

(12) **EUROPÄISCHE PATENTSCHRIFT**

(45) Veröffentlichungstag der Patentschrift :
20.10.93 Patentblatt 93/42

(21) Anmeldenummer : 91900786.4

(22) Anmeldetag : 19.12.90

(86) Internationale Anmeldenummer :
PCT/EP90/02252

(87) Internationale Veröffentlichungsnummer :
WO 91/08767 27.06.91 Gazette 91/14

(51) Int. Cl.⁵ : **A61K 37/54**, A61K 37/547,
A61K 47/12, A61K 47/18,
A61K 47/22, A61K 47/26,
A61K 47/20

(54) **STABILISIERUNG VON GLYKOSYLIERTEM t-PA.**

(30) Priorität : 20.12.89 DE 3942142

(43) Veröffentlichungstag der Anmeldung :
04.12.91 Patentblatt 91/49

(45) Bekanntmachung des Hinweises auf die
Patenterteilung :
20.10.93 Patentblatt 93/42

(84) Benannte Vertragsstaaten :
AT BE CH DE DK ES FR GB GR IT LI LU NL SE

(56) Entgegenhaltungen :
EP-A- 0 156 169
EP-A- 0 211 592
EP-A- 0 217 379
EP-A- 0 228 862
EP-A- 0 297 294
WO-A-90/01333

(73) Patentinhaber : **BOEHRINGER MANNHEIM GMBH**
**D-68298 Mannheim (DE)**

(72) Erfinder : **KOHNERT, Ulrich, Dr.**
**Heubachweg 6**
**W-8121 Habach (DE)**
Erfinder : **RUDOLPH, Rainer, Dr.**
**Färbergasse 19**
**W-8120 Weilheim (DE)**

(74) Vertreter : **Huber, Bernhard, Dipl.-Chem. et al**
**Patentanwälte H. Weickmann, Dr. K. Fincke**
**F.A. Weickmann, B. Huber Dr. H. Liska, Dr. J.**
**Prechtel Kopernikusstrasse 9 Postfach 86 08 20**
**D-81635 München (DE)**

EP 0 458 930 B1

## Beschreibung

Der menschliche Gewebe-Plasminogen-Aktivator (t-PA) besitzt eine große therapeutische Bedeutung bei der Auflösung von Blutgerinnseln, z.B. bei Herzinfarkten. t-PA bewirkt die Auflösung von Blutgerinnseln durch die Aktivierung von Plasminogen zu Plasmin. Plasmin wiederum löst Fibrin, die Hauptkomponente der Proteinmatrix von geronnenem Blut.

Natürlicher t-PA ist aus mehreren funktionellen Domänen F, E, K1, K2 und P zusammengesetzt. Die Domäne P beinhaltet das proteolytisch aktive Zentrum, das die Spaltung von Plasminogen zu Plasmin bewirkt. Die gentechnologische Herstellung von t-PA oder verschiedenen t-PA-Muteinen, bei denen eine oder mehrere der Domänen F, E, K1 und K2 deletiert sind, in eukaryontischen und prokaryontischen Zellen ist bereits bekannt. Dabei werden t-PA-Derivate aus Prokaryonten im Gegensatz zu natürlichem t-PA in nicht glykosylierter Form synthetisiert.

Glykosylierte Proteine mit t-PA-Aktivität lösen sich nur in geringer Konzentration in den üblicherweise zur Solubilisierung von Proteinen verwendeten Puffern, wie z.B. 50 mmol/l Na-Citrat, 50 mmol/l Phosphat oder physiologische NaCl-Lösung. Für den Einsatz als therapeutischer Wirkstoff sollten jedoch Proteinlösungen mit einer höheren t-PA-Aktivität von mindestens 1,4 MU/ml, vorzugsweise 1,4 MU/ml bis 10 MU/ml vorliegen.

In EP-A 0 297 294 wird gelehrt, dem t-PA mindestens zwei Substanzen aus der Gruppe der D- und/oder L-Aminosäuren zuzusetzen Auch EP-A o 228 862 und EP-A 0 217 379 lehren einen Zusatz von Arginin bei jeweils unterschiedlichen Chloridionenkonzentrationen. Bei der Erfindung werden jedoch Zusätze von Aminosäuren nicht in Betracht gezogen. Dies gilt auch für die EP-A 0 156 169, wo L-Lysin als Zusatz offenbart wird. Aus EP-A 0 211 592 schließlich ist es bekannt, tPA in einer gepufferten Lösung bei pH 3,5 bis 5,5 in Abwesenheit von löslichkeitsverbessernden und stabilisierenden Mitteln einzusetzen. All diesen Vorschlägen ist gemeinsam, daß sie nicht die angestrebten Aktivitätskonzentrationen zu erreichen gestatten, nämlich Aktivitäten von mindestens 1,4 MU/ml.

Das US-Patent 4,777,043 offenbart eine pharmazeutische Zusammensetzung mit menschlichem t-PA und einem pharmazeutisch verträglichen Argininiumionen enthaltenden Puffer mit einer Chloridionen-Konzentration bis 0,3 mol/l. Die EP-A 0 303 351 offenbart weitere Möglichkeiten, Proteine mit t-PA-Aktivität in Puffern durch bestimmte Aminosäuren, deren Salze, Derivate und Homologe zu solubilisieren. Weiterhin kann t-PA durch Zusatz von Gelatine gemäß EP-A 0 123 304, durch Zusatz von Albumin gemäß EP-A 0 112 940 oder durch Zugabe eines Polyschwefelsäureesters eines Saccharids oder eines sulfatierten Zuckers gemäß EP-A 0 198 321 stabilisiert werden. Die PCT/US88/04402 offenbart ein Verfahren zur Erhöhung der t-PA-Löslichkeit, worin man ein wäßriges Medium mit einer basischen Aminosäure, insbesondere Arginin, in einer Konzentration von 0,02 bis 0,2 mol/l zusammen mit einer Citronensäuregruppe in einer Konzentration von 0,02 bis 0,08 mol/l bei einem pH-Wert von 5 bis 8 verwendet.

Diese verschiedenen Zusammensetzungen sind jedoch nicht generell für alle Proteine mit t-PA-Eigenschaften geeignet. So wurde festgestellt, daß verschiedene glykosylierte bzw. nicht glykosylierte t-PA-Varianten stark voneinander abweichende Lösungseigenschaften besitzen.

Aufgabe der Erfindung war es demnach, pharmazeutische Präparate zu entwickeln, die insbesondere glykosylierten t-PA bzw. t-PA-Muteine mit einer Aktivität von mehr als 1,4 MU/ml enthalten, wobei der t-PA über einen längeren Zeitraum hinweg stabil sein soll. Die Einheit U ist gemäß WHO, National Institute for Biological Standards and Control (vgl. H. Lill, ZGIMAL 42 (1987), 478-486) definiert.

Die erfindungsgemäße Aufgabe wird gelöst durch ein pharmazeutisches Präparat eines glykosylierten Proteins mit t-PA-Aktivität mit einer Aktivität von mindestens 1,4 MU/ml mit einem pH-Wert von 4,5 bis 9, wobei diese Zusammensetzung Citrat und mindestens eine Verbindung aus der aus

a) Ascorbinsäure,
b) EDTA,
c) Aminoverbindungen der Formel

$$R^1R^2N - R - X$$

wobei X = $SO_3H$, H, $NH_2$ oder OH ist, R = $C_1$-$C_9$-Alkylen, $C_3$-$C_6$-Cycloalkylen oder Benzyliden ist und $R^1$ und $R^2$ voneinander unabhängig H oder $C_1$-$C_3$-Alkyl sind,

d) Guanidinobuttersäure,
e) Dimethylbiguanid,
f) 7-Aminoheptansäure, 8-Aminooctansäure, p-Aminomethylbenzoesäure, δ-Aminovaleriansäure, γ-Aminobuttersäure,
g) Glucosamin, Fructose,
h) Pyrimidinnukleosiden und Pyrimidinnukleotiden,
i) Apfelsäure, Milchsäure, Fumarsäure oder 2-Oxoglutarsäure

bestehenden Gruppe enthält zusammen mit t-PA oder einem t-PA-Mutein, bei dem eine oder mehrere der Do-

2

mänen F, E, $K_1$ und $K_2$ deletiert sind.

Die Konzentration der Citrationen in einem erfindungsgemäßen pharmazeutischen Präparat soll mindestens 5 mmol/l, vorzugsweise von 5 bis 100 mmol/l betragen, besonders bevorzugt ist eine Konzentration der Citrationen von 50 mmol/l.Der pH-Wert wird je nach Basizität der zugesetzten Verbindung vorzugsweise mit HCl oder einer Base wie z.B. NaOH oder KOH eingestellt.

Es hat sich als geeignet erwiesen, den pH-Wert der alkalischen Citrat-Lösungen mit HCl einzustellen, d.h. daß die Zusammensetzung zusätzlich noch Chloridionen enthält. In Gegenwart von Chloridionen sind nämlich überraschenderweise hochkonzentrierte Lösungen von t-PA bzw. t-PA-Derivaten wesentlich stabiler als z.B. in Gegenwart von Phosphationen. Der pH-Wert von sauren Citrat-Lösungen wird üblicherweise mit NaOH eingestellt.

Geeignet für eine erfindungsgemäße Zusammensetzung ist ein pH-Wert zwischen 4,5 und 9, bevorzugt ist ein pH-Wert von 6.

Unter einem glykosylierten Protein mit t-PA-Aktivität gemäß vorliegender Erfindung sind nicht modifizierter t-PA aus eukaryontischen Organismen, sowie alle glykosylierten t-PA-Muteine zu verstehen. Beispiele für t-PA-Muteine sind z.B. bei Harris (Protein Engineering 1 (1987), 449-458) beschrieben.

Vorzugsweise enthält die erfindungsgemäße Zusammensetzung nativen glykosylierten t-PA mit den Domänen F, E, K1, K2 und P aus CHO-Zellen (Herstellung nach WO 87/02673). Geeignet sind jedoch auch alle anderen t-PA-Varianten aus Eukaryonten.

Vorzugsweise werden für eine erfindungsgemäße Zusammensetzung als Aminoverbindungen Taurin, 4-Aminobutanol-1, 5-Aminopentanol-1, 6-Aminohexanol-1, 1,9-Diaminononan, 1,8-Diaminooctan, 1,7-Diaminoheptan, 1,6-Diaminohexan, 1,5-Diaminopentan, 1,4-Aminobutan oder 1,3-Aminopropan verwendet. Die bevorzugte Konzentration für Taurin und analoge Verbindungen beträgt 0,1 bis 0,5 mol/l, besonders bevorzugt 0,1 bis 0,3 mol/l. Die anderen obengenannten Verbindungen ($\alpha,\omega$-Diamine und $\alpha,\omega$-Aminoalkohole) werden vorzugsweise mit 10 bis 100 mmol/l eingesetzt.

Apfelsäure, Milchsäure, Fumarsäure oder 2-Oxoglutarsäure werden vorzugsweise mit 1 mmol/l bis 1000 mmol/l, besonders bevorzugt mit 10 bis 500 mmol/l verwendet.

Guanidinobuttersäure wird vorzugsweise mit 10 bis 200 mmol/l, besonders bevorzugt mit 50 bis 100 mmol/l verwendet. Für Dimethylbiguanid beträgt die Konzentration 50 bis 400 mmol/l, vorzugsweise 100 bis 300 mmol/l.

7-Aminoheptansäure, 8-Aminooctansäure, $\delta$-Aminovaleriansäure, $\gamma$-Aminobuttersäure oder p-Aminomethylbenzoesäure verwendet man bevorzugt mit 0,5 bis 20 mmol/l, besonders bevorzugt mit 1 bis 10 mmol/l. Diese Substanzen bewirken überraschenderweise bereits in geringem molaren Überschuß (10- bis 40fach) eine hervorragende Löslichkeit von glykosylierten t-PA-Derivaten.

Glucosamin und Fructose werden bevorzugt in Konzentrationen von 1 bis 500 mmol/l, besonders bevorzugt mit 10 bis 300 mmol/l eingesetzt.

Als Pyrimidinnukleosid oder Pyrimidinnukleotid sind z.B. Thymidin, Cytosin und Uridin bzw. die entsprechenden Nukleotide geeignet. Diese Substanzen werden vorzugsweise in Konzentrationen von 1 bis 300 mmol/l, besonders bevorzugt 10 bis 300 mmol/l eingesetzt.

Weiterhin ein Gegenstand der Erfindung ist eine erfindungsgemäße Zusammensetzung, die zusätzlich eine oder mehrere Aminosäuren, insbesondere Histidin enthält.

Im folgenden ist eine Reihe besonders bevorzugter Präparate gemäß vorliegender Erfindung aufgeführt.

Eine Formulierung enthält 50 mmol/l Na-Citrat, pH 6 und 0,1 bis 0,3 mol/l Taurin. Bevorzugt ist auch eine Formulierung mit 50 mmol/l Na-Citrat/NaOH, pH 6 und 0,2 bis 0,3 mol/l Ascorbinsäure.

Weiterhin bevorzugt ist eine Formulierung mit 50 mmol/l Na-Citrat/HCl, pH 6 und 1 mmol/l bis 10 mmol/l 7-Aminoheptansäure, 8-Aminooctansäure, $\delta$-Aminovaleriansäure, $\gamma$-Aminobuttersäure oder p-Aminomethylbenzoesäure.

Weiterhin besonders bevorzugt sind auch Formulierungen, die 50 mmol/l Na-Citrat/HCl, pH 6,0 und 50 bis 100 mmol/l Guanidinobuttersäure enthalten.

Bevorzugt ist auch eine Formulierung, die 50 mmol/l Na-Citrat, pH 6 und 10 bis 100 mmol/l EDTA enthält. Wiederum eine weitere Formulierung enthält 50 mmol/l NaCitrat/HCl, pH 6 und 100 bis 300 mmol/l Dimethylbiguanid.

Eine weitere Formulierung enthält 50 mmol/l NaCitrat, pH 6 und 10 bis 300 mmol/l Thymidin, Cytosin oder Uridin.

Wiederum eine weitere Formulierung enthält 50 mmol/l NaCitrat/HCl, pH 6 und 10 bis 100 mmol/l 4-Aminobutanol-1, 5-Aminopentanol-1, 6-Aminohexanol-1, 1,9-Diaminononan, 1,8-Diaminooctan, 1,7-Diaminoheptan, 1,6-Diaminohexan, 1,5-Diaminopentan, 1,4-Aminobutan oder 1,3-Aminopropan.

Eine weitere Formulierung enthält 50 mmol/l NaCitrat/HCl, pH 6 und 10 bis 300 mmol/l Fructose oder Glucosamin.

Schließlich enthält eine weitere Formulierung 50 mmol/l NaCitrat, pH 6 und 10 bis 500 mmol/l Apfelsäure, Milchsäure, Fumarsäure oder 2-Oxoglutarsäure.

Auch Kombinationen aus mehreren der obengenannten Verbindungen mit Citrat bewirken eine sehr gute Löslichkeit von glykosylierten Proteinen mit t-PA-Aktivität, insbesondere von t-PA aus CHO-Zellen.

Ein Gegenstand der Erfindung ist auch ein Arzneimittel auf Basis eines glykosylierten Proteins mit t-PA-Aktivität als Wirkstoff in Lösung oder als Lyophilisat mit den angegebenen Wirkstoffen und gegebenenfalls noch weiteren pharmazeutisch verträglichen Zusatz-, Hilfs-, Träger- und Füllstoffen.

Die erfindungsgemäßen pharmazeutischen Präparate kommen vorzugsweise als Injektions- und Infusionslösungen zum Einsatz. Dies kann dadurch geschehen, daß eine bereits spritzfertige Lösung zur Verfügung gestellt wird, die die erfindungsgemäße Zusammensetzung besitzt. Es ist jedoch auch möglich, die pharmazeutischen Präparate in Form von Lyophilisaten zur Verfügung zu stellen. Diese werden dann mit an sich bekannten, zu Injektionszwecken geeigneten Mitteln oder Lösungen rekonstituiert. Als Injektionsmedium kommt vorzugsweise Wasser zur Anwendung, welches die bei Injektionslösungen üblichen Zusätze wie Stabilisierungsmittel, Lösungsvermittler, Puffer und isotonische Zusätze, beispielsweise eine physiologische NaCl-Konzentration, enthält. Derartige Zusätze sind beispielsweise Mannit, Tartrat- oder Citrat-Puffer, Ethanol, Komplexbildner wie z.B. Ethylendiamintetraessigsäure und deren nicht-toxischen Salze, sowie hochmolekulare Polymere, wie flüssiges Polyethylenoxid zur Viskositätsregulierung. Flüssige Trägerstoffe für Injektionslösungen müssen steril sein und werden vorzugsweise in Ampullen abgefüllt.

Schließlich beinhaltet die vorliegende Erfindung auch die Verwendung von glykosylierten Proteinen mit t-PA-Aktivität zur Herstellung von erfindungsgemäßen pharmazeutischen Präparaten.

Das folgende Beispiel soll die konkrete Ausführung der Erfindung weiter erläutern.

**Beispiel**

**Löslichkeit von CHO-tPA**

Gereinigtes CHO-tPA (gelöst in 0.5 mol/l Arginin/$H_3PO_4$, pH 7,3) wird durch Ultrafiltration über eine YM 10-Membran (Amicon®) konzentriert. Jeweils 1 ml des Konzentrats (Aktivität: 6L, 6 MU/ml) wird gegen die in Tabelle 1 aufgeführten Puffer dialysiert. Nach Zentrifugation der Proben wird im klaren Überstand die enzymatische Aktivität gemessen.

Die enzymatische Aktivität ist als Volumeneinheit in MU/ml und als Gesamtaktivität in MU angegeben.

Die Messung der tPA-Aktivität kann dabei auf übliche Weise durch Spaltung eines chromogenen Substrats bestimmt werden (H. Lill, ZGIMAL 42, (1987), 478 - 486).

Die Einheit U ist eine Einheit der Aktivität nach Definition der WHO, National Institute for Biological Standards and Control.

T a b e l l e    1

| Puffer | Aktivität | |
| --- | --- | --- |
| | MU/ml | MU |
| 50 mmol/l NaCitrat/NaOH, pH 6<br>0,3 mol/l Taurin | 4,60 | 5,52 |
| 50 mmol/l NaCitrat/HCl, pH 6<br>0,3 mol/l Fructose | 1,65 | 1,73 |
| 50 mmol/l Nacitrat/NaOH, pH 6<br>0,3 mol/l Ascorbinsäure | 4,76 | 4,76 |
| 50 mmol/l NaCitrat/HCl, pH 6<br>10 mmol/l p-Aminomethyl-<br>benzoesäure | 5,58 | 6.40 |
| 50 mmol/l NaCitrat/HCl, pH 6<br>300 mmol/l Dimethylbiguanid | 5,91 | 6,50 |
| 0,05 mol/l Tris/HCl, pH 7.2 | 0,02 | 0,04 |
| 50 mmol/l $NH_4 HCO_3$ | 0,06 | 0,09 |
| 50 mmol/l $Na_2 HPO_4 /H_3 PO_4$, pH 6 | 0,14 | 0,19 |

| Puffer | Aktivität | |
|---|---|---|
| | MU/ml | MU |
| 50 mmol/l NaCitrat/HCl, pH 6 10 mmol/l 7-Aminoheptansäure | 5,07 | 7,00 |
| 50 mmol/l NaCitrat/HCl, pH 6 10 mmol/l δ-Aminovaleriansäure | 4,00 | 4,80 |
| 50 mmol/l NaCitrat/HCl, pH 6 10 mmol/l γ-Aminobuttersäure | 3,18 | 4,13 |
| 50 mmol/l NaCitrat/HCl, pH 6 50 mmol/l 1,6-Diaminohexan | 5,20 | 6,30 |
| 50 mmol/l NaCitrat/HCl, pH 6 50 mmol/l 5-Aminopentanol | 6,00 | 6,00 |
| 50 mmol/l Nacitrat/HCl, pH 6 50 mmol/l Guanidinobuttersäure | 3,24 | 3,90 |
| 50 mmol/l NaCitrat/NaOH, pH 6 50 mmol/l EDTA | 3,42 | 3,76 |
| 50 mmol/l NaCitrat/NaOH, pH 6 100 mmol/l EDTA | 5,07 | 5,8 |
| 50 mmol/l NaCitrat/HCl, pH 6 0,3 mol/l Glucosamin | 3,42 | 3,76 |

| Puffer | Aktivität | |
|---|---|---|
| | MU/ml | MU |
| 50 mmol/l Nacitrat/HCl, pH 6<br>0,1 mol/l Thymidin | 2,40 | 2,88 |
| 50 mmol/l NaCitrat/HCl, pH 6 | 0,79 | 0,98 |
| 50 mmol/l NaCitrat/NaOH, pH 6<br>0,3 mol/l Fumarsäure | 2,97 | 2,97 |

**Patentansprüche**

1. Pharmazeutisches Präparat eines glykosylierten Proteins mit t-PA-Aktivität mit einer Aktivität von mindestens 1,4 MU/ml und einem pH-Wert von 4,5 bis 9,
   **dadurch gekennzeichnet,**
   daß es Citrat und mindestens eine Verbindung aus der aus
   a) Ascorbinsäure,
   b) EDTA,
   c) Aminoverbindungen der Formel
   $$R^1R^2N - R - X$$
   wobei X = $SO_3H$, H, $NH_2$ oder OH ist, R = $C_1$-$C_9$-Alkylen, $C_3$-$C_6$-Cycloalkylen oder Benzyliden ist und $R^1$ und $R^2$ voneinander unabhängig H oder $C_1$-$C_3$-Alkyl sind,
   d) Guanidinobuttersäure,
   e) Dimethylbiguanid,
   f) 7-Aminoheptansäure, 8-Aminooctansäure, p-Aminomethylbenzoesäure, δ-Aminovaleriansäure, γ-Aminobuttersäure,
   g) Glucosamin, Fructose,
   h) Pyrimidinnukleosiden und Pyrimidinnukleotiden,
   i) Apfelsäure, Milchsäure, Fumarsäure oder 2-Oxoglutarsäure bestehenden Gruppe enthält zusammen mit t-PA oder einem t-PA-Mutein, bei dem eine oder mehrere der Domänen F, E, $K_1$ und $K_2$ deletiert sind.

2. Pharmazeutisches Präparat nach Anspruch 1,
   **dadurch gekennzeichnet,**
   daß die Aminoverbindung Taurin, 4-Aminobutanol-1, 5-Aminopentanol-1, 6-Aminohexanol-1, 1,9-Diaminononan, 1,8-Diaminooctan, 1,7-Diaminoheptan, 1,6-Diaminohexan, 1,5-Diaminopentan, 1,4-Diaminobutan oder 1,3-Diaminopropan ist.

3. Pharmazeutisches Präparat nach Anspruch 1,
   **dadurch gekennzeichnet,**
   daß es zusätzlich eine oder mehrere Aminosäuren enthält.

4. Pharmazeutisches Präparat nach einem der Ansprüche 1 bis 4, **dadurch**

7

**gekennzeichnet,** daß die Citrat-Konzentration 5 bis 100 mmol/l, vorzugsweise 50 mmol/l beträgt.

5. Pharmazeutisches Präparat nach einem der Ansprüche 1 bis 5, **dadurch gekennzeichnet,** daß es zusätzlich Chlorid-Ionen enthält.

6. Pharmazeutisches Präparat nach Anspruch 1,
**dadurch gekennzeichnet,**
daß es 50 mmol/l NaCitrat, pH 6, und 0,1 bis 1 mol/l, vorzugsweise 0,2 bis 0,3 mol/l Ascorbinsäure enthält.

7. Pharmazeutisches Präparat nach Anspruch 1,
**dadurch gekennzeichnet,**
daß es 50 mmol/l NaCitrat, pH 6, und 1 bis 200 mmol/l, vorzugsweise 10 bis 100 mmol/l EDTA enthält.

8. Pharmazeutisches Präparat nach Anspruch 1,
**dadurch gekennzeichnet,**
daß es 50 mmol/l NaCitrat, pH 6, und 0,1 bis 0,5 mol/l, vorzugsweise 0,1 bis 0,3 mol/l Taurin enthält.

9. Pharmazeutisches Präparat nach Anspruch 1,
**dadurch gekennzeichnet,**
daß es 50 mmol/l NaCitrat/HCl, pH 6, und 10 bis 100 mmol/l 4-Aminobutanol-1, 5-Aminopentanol-1, 6-Aminohexanol-1, 1,3-Diaminopropan, 1,4-Diaminobutan, 1,5-Diaminopentan, 1,6-Diaminohexan, 1,7-Diaminoheptan, 1,8-Diaminooctan oder 1,9-Diaminononan enthält.

10. Pharmazeutisches Präparat nach Anspruch 1,
**dadurch gekennzeichnet,**
daß es 50 mmol/l NaCitrat/HCl pH 6, und 10 bis 200 mmol/l, vorzugsweise 50 bis 100 mmol/l Guanidinobuttersäure enthält.

11. Pharmazeutisches Präparat nach Anspruch 1,
**dadurch gekennzeichnet,**
daß es 50 mmol/l NaCitrat/HCl und 50 bis 400 mmol/l, vorzugsweise 100 bis 300 mmol/l Dimethylbiguanid enthält.

12. Pharmazeutisches Präparat nach Anspruch 1,
**dadurch gekennzeichnet,**
daß es 50 mmol/l NaCitrat/HCl, pH 6, und 0,5 bis 20 mmol/l, vorzugsweise 1 bis 10 mmol/l 7-Aminoheptansäure, 8-Aminooctansäure, δ-Aminovaleriansäure, γ-Aminobuttersäure oder p-Aminomethylbenzoesäure enthält.

13. Pharmazeutisches Präparat nach Anspruch 1,
**dadurch gekennzeichnet,**
daß es 50 mmol/l NaCitrat/HCl, pH 6, und 1 bis 500 mmol/l, vorzugsweise 10 bis 300 mmol/l Glucosamin oder Fructose enthält.

14. Pharmazeutisches Präparat nach Anspruch 1,
**dadurch gekennzeichnet,**
daß es 50 mmol/l NaCitrat/HCl, pH 6, und 1 bis 300 mmol/l, vorzugsweise 10 bis 300 mmol/l Thymidin, Cytosin oder Uridin enthält.

15. Pharmazeutisches Präparat nach Anspruch 1,
**dadurch gekennzeichnet,**
daß es 50 mmol/l NaCitrat, pH 6, und 1 bis 1000 mmol/l, vorzugsweise 10 bis 500 mmol/l Apfelsäure, Milchsäure, Fumarsäure oder 2-Oxoglutarsäure enthält.

16. Pharmazeutisches Präparat nach Anspruch 1,
**dadurch gekennzeichnet,**
daß es 50 mmol/l NaCitrat, pH 6, und eine Kom- bination von Substanzen aus der Gruppe a) bis i) nach Anspruch 1 enthält.

17. Pharmazeutisches Präparat auf Basis eines glykosylierten Proteins mit t-PA-Aktivität als Wirkstoff,

**gekennzeichnet durch**
die Zusammensetzung nach einem der vorhergehenden Ansprüche, gegebenenfalls zusammen mit üblichen pharmazeutischen Zusatz-, Hilfs- oder/und Trägerstoffen.

18. Verfahren zur Herstellung eines pharmazeutischen Präparats nach einem der Ansprüche 1 bis 17, **dadurch gekennzeichnet,** daß man ein glykosyliertes Protein mit t-PA-Aktivität zusammen mit mindestens einer Substanz aus der Gruppe a) bis i) nach Anspruch 1 in eine geeignete pharmazeutische Darreichungsform überführt.

19. Verfahren nach Anspruch 18, **dadurch gekennzeichnet,** daß die pharmazeutische Darreichungsform eine Injektionslösung oder ein Lyophilisat ist.

20. Verwendung eines glykosylierten Proteins mit t-PA-Aktivität zur Herstellung von pharmazeutischen Präparaten nach einem der Ansprüche 1 bis 17.


## Claims

1. Pharmaceutical preparation of a glycosated protein with t-PA activity with an activity of at least 1.4 MU/ml and a pH value of 4.5 to 9, characterised in that it contains citrate and at least one compound from the group consisting of
    a) ascorbic acid,
    b) EDTA,
    c) amino compounds of the formula
$$R^1R^2N - R - X$$
whereby X = $SO_3H$, H, $NH_2$ or OH, R = $C_1$-$C_9$-alkylene, $C_3$-$C_6$-cycloalkylene or benzylidene and $R^1$ and $R^2$, independently of one another, are H or $C_1$-$C_3$-alkyl,
    d) guanidinobutyric acid,
    e) dimethylbiguanide,
    f) 7-aminoheptanoic acid, 8-aminooctanoic acid, p-aminomethylbenzoic acid, $\delta$-aminovaleric acid, $\gamma$-aminobutyric acid,
    g) glucosamine, fructose,
    h) pyrimidine nucleosides and pyrimidine nucleotides,
    i) malic acid, lactic acid, fumaric acid or 2-oxoglutaric acid,
together with t-PA or a t-PA mutein in which one or more of the domains F, E, $K_1$ and $K_2$ are deleted.

2. Pharmaceutical preparation according to claim 1, characterised in that the amino compound is taurine, 4-aminobutanol-1, 5-aminopentanol-1, 6-aminohexanol-I, 1,9-diaminononane, 1,8-diaminooctane, 1,7-diaminoheptane, 1,6-diaminohexane, 1,5-diaminopentane, 1,4-diaminobutane or 1,3-diaminopropane.

3. Pharmaceutical preparation according to claim 1, characterised in that it additionally contains one or more amino acids.

4. Pharmaceutical preparation according to one of claims 1 to 3, characterised in that the citrate concentration amounts to 5 to 100 mmole/l, preferably 50 mmole/l.

5. Pharmaceutical preparation according to one of claims 1 to 4, characterised in that it additionally contains chloride ions.

6. Pharmaceutical preparation according to claim 1, characterised in that it contains 50 mmole/l Na citrate, pH 6, and 0.1 to 1 mole/l, preferably 0.2 to 0.3 mole/l, ascorbic acid.

7. Pharmaceutical preparation according to claim 1, characterised in that it contains 50 mmole/l Na citrate, pH 6, and 1 to 200 mmole/l, preferably 10 to 100 mmole/l EDTA.

8. Pharmaceutical preparation according to claim 1, characterised in that it contains 50 mmole/l Na citrate, pH 6, and 0.1 to 0.5 mole/l, preferably 0.1 to 0.3 mole/l taurine.

9. Pharmaceutical preparation according to claim 1, characterised in that it contains 50 mmole/l Na citrate/HCl, pH 6, and 10 to 100 mmole/l 4-aminobutanol-l, 5-aminopentanol-1, 6-aminohexanol-1, 1,3-diaminopropane, 1,4-diaminobutane, 1,5-diaminopentane, 1,6-diaminohexane, 1,7-diaminoheptane, 1,8-diaminooctane or 1,9-diaminononane.

10. Pharmaceutical preparation according to claim 1, characterised in that it contains 50 mmole/l Na citrate/HCl, pH 6, and 10 to 200 mmole/l, preferably 50 to 100 mmole/l guanidinobutyric acid.

11. Pharmaceutical preparation according to claim 1, characterised in that it contains 50 mmole/l Na citrate/HCl and 50 to 400 mmole/l, preferably 100 to 300 mmole/l dimethylbiguanide.

12. Pharmaceutical preparation according to claim 1, characterised in that it contains 50 mmole/l Na citrate/HCl, pH 6, and 0.5 to 20 mmole/l, preferably 1 to 10 mmole/l 7-aminoheptanoic acid, 8-aminooctanoic acid, δ-aminovaleric acid, γ-aminobutyric acid or p-aminomethylbenzoic acid.

13. Pharmaceutical preparation according to claim 1, characterised in that it contains 50 mmole/l Na citrate/HCl, pH 6, and 1 to 500 mmole/l, preferably 10 to 300 mmole/l glucosamine or fructose.

14. Pharmaceutical preparation according to claim 1, characterised in that it contains 50 mmole/l Na citrate/HCl, pH 6, and 1 to 300 mmole/l, preferably 10 to 300 mmole/l thymidine, cytosine or uridine.

15. Pharmaceutical preparation according to claim 1, characterised in that it contains 50 mmole/l Na citrate, pH 6, and 1 to 1000 mmole/l, preferably 10 to 500 mmole/l malic acid, lactic acid, fumaric acid or 2-oxoglutaric acid.

16. Pharmaceutical preparation according to claim 1, characterised in that it contains 50 mmole/l Na citrate, pH 6, and a combination of substances from the group a) to i) according to claim 1.

17. Pharmaceutical preparation based on a glycosylated protein with t-PA activity as active material, characterised by the composition according to one of the preceding claims, possibly together with usual pharmaceutical additive, adjuvant and/or carrier materials.

18. Process for the preparation of a pharmaceutical composition according to one of claims 1 to 17, characterised in that one converts a glycosylated protein with t-PA activity, together with at least one substance from the group a) to i) according to claim 1, into a suitable pharmaceutical form of administration.

19. Process according to claim 18, characterised in that the pharmaceutical form of administration is an injection solution or a lyophilisate.

20. Use of a glycosylated protein with t-PA activity for the preparation of pharmaceutical preparations according to one of claims 1 to 17.

**Revendications**

1. Préparation pharmaceutique d'une protéine glycosylée à activité t-PA présentant une activité d'au moins 1,4 MU/ml et un pH de 4,5 à 9, caractérisée en ce qu'elle contient du citrate et au moins un composé du groupe formé par

a) l'acide ascorbique,

b) l'EDTA

c) un composé aminé de formule

$$R^1R^2N - R - X$$

dans laquelle X = $SO_3H$, H, $NH_2$ ou OH, R = alkyléne en $C_1$ - $C_9$, cycloalkylène en $C_3$ - $C_6$ ou benzylidène et $R^1$ et $R^2$ sont indépendamment l'un de l'autre H ou alkyle en $C_1$ - $C_3$,

d) l'acide guanidinobutyrique,

e) le diméthylbiguanide,

f) l'acide 7-aminoheptanoïque, l'acide 8-aminooctanoïque, l'acide p-aminométhylbenzoïque, l'acide δ-aminovalérique, l'acide γ-aminobutyrique,

g) la glucosamine, le fructose,

h) des nucléosides pyrimidiques et des nucléotides pyrimidiques,

i) l'acide malique, l'acide lactique, l'acide fumarique ou l'acide 2-oxoglutarique

ainsi que le t-PA ou une mutéine du t-PA dans laquelle un ou plusieurs des domaines F, E, $K_1$ et $K_2$ sont délétés.

2. Préparation pharmaceutique selon la revendication 1, caractérisée en ce que le composé aminé est la taurine, le 4-aminobutanol-1, le 5-aminopentanol-1, le 6-aminohexanol-1, le 1,9-diaminononane, le 1,8-diamino-octane, le 1,7-diaminoheptane, le 1,6-diaminohexane, le 1,5-diaminopentane, le 1,4-diaminobutane ou le 1,3-diaminopropane.

3. Préparation pharmaceutique selon la revendication 1, caractérisée en ce qu'elle contient en outre un ou plusieurs acides aminés.

4. Préparation pharmaceutique selon l'une des revendications 1 à 3, caractérisée en ce que la concentration du citrate est de 5 à 100 mmol/l, de préférence de 50 mmol/l.

5. Préparation pharmaceutique selon l'une des revendications 1 à 4, caractérisée en ce qu'elle contient en outre des ions chlorure.

6. Préparation pharmaceutique selon la revendication 1, caractérisée en ce qu'elle contient 50 mmol/l de citrate de Na, pH 6, et 0,1 à 1 mol/l, de préférence 0,2 à 0,3 mol/l d'acide ascorbique.

7. Préparation pharmaceutique selon la revendication 1, caractérisée en ce qu'elle contient 50 mmol/l de citrate de Na, pH 6, et 1 à 200 mmol/l, de préférence 10 à 100 mmol/l de EDTA.

8. Préparation pharmaceutique selon la revendication 1, caractérisée en ce qu'elle contient 50 mmol/l de citrate de Na, pH 6, et 0,1 à 0,5 mol/l, de préférence 0,1 à 0,3 mol/l de taurine.

9. Préparation pharmaceutique selon la revendication 1, caractérisée en ce qu'elle contient 50 mmol/l de citrate de Na/HCl, pH 6, et 10 à 100 mmol/l de 4-aminobutanol-1, de 5-aminopentanol-1, de 6-aminohexanol-1, de 1,3-diaminopropane, de 1,4-diaminobutane, de 1,5-diaminopentane, de 1,6-diaminohexane, de 1,7-diaminoheptane, de 1,8-diaminooctane ou de 1,9-diaminononane.

10. Préparation pharmaceutique selon la revendication 1, caractérisée en ce qu'elle contient 50 mmol/l de citrate de Na/HCl, pH 6, et 10 à 200 mmol/l, de préférence 50 à 100 mmol/l d'acide guanidinobutyrique.

11. Préparation pharmaceutique selon la revendication 1, caractérisée en ce qu'elle contient 50 mmol/l de citrate de Na/HCl et 50 à 400 mmol/l, de préférence 100 à 300 mmol/l de diméthylbiguanide.

12. Préparation pharmaceutique selon la revendication 1, caractérisée en ce qu'elle contient 50 mmol/l de citrate de Na/HCl, pH 6, et 0,5 à 20 mmol/l, de préférence 1 à 10 mmol/l d'acide 7-aminoheptanoïque, d'acide 8-aminooctanoïque, d'acide $\delta$-aminovalérique, d'acide $\gamma$-aminobutyrique ou d'acide p-aminométhylbenzoïque.

13. Préparation pharmaceutique selon la revendication 1, caractérisée en ce qu'elle contient 50 mmol/l de citrate de Na/HCl, pH 6, et 1 à 500 mmol/l, de préférence 10 à 300 mmol/l de glucosamine ou de fructose.

14. Préparation pharmaceutique selon la revendication 1, caractérisée en ce qu'elle contient 50 mmol/l de citrate de Na/HCl, pH 6, et 1 à 300 mmol/l, de préférence 10 à 300 mmol/l de thymidine, de cytosine ou d'uridine.

15. Préparation pharmaceutique selon la revendication 1, caractérisée en ce qu'elle contient 50 mmol/l de citrate de Na, pH 6, et 1 à 1000 mmol/l, de préférence 10 à 500 mmol/l d'acide malique, d'acide lactique, d'acide fumarique ou d'acide 2-oxoglutarique.

16. Préparation pharmaceutique selon la revendication 1, caractérisée en ce qu'elle contient 50 mmol/l de citrate de Na, pH 6, et une combinaison de substances du groupe a) à i) selon la revendication 1.

17. Préparation pharmaceutique à base d'une protéine glycosylée à activité t-PA en tant que principe actif, caractérisée par la composition selon l'une des revendications précédentes, éventuellement avec des additifs, adjuvants et/ou véhicules pharmaceutiques habituels.

18. Procédé d'obtention d'une préparation pharmaceutique selon l'une des revendications 1 à 17, caractérisé en ce que l'on convertit en une forme d'administration pharmaceutique appropriée une protéine glycosylée à activité t-PA et au moins une substance du groupe a) à i) selon la revendication 1.

19. Procédé selon la revendication 18, caractérisé en ce que la forme d'administration pharmaceutique est une solution pour injection ou un lyophilisat.

20. Utilisation d'une protéine glycosylée à activité t-PA pour l'obtention de préparations pharmaceutiques selon l'une des revendications 1 à 17.